(19) Europäisches Patentamt
European Patent Office
Office européen des brevets

(11) Publication number: **0 214 112 B1**

(12) # EUROPEAN PATENT SPECIFICATION

(45) Date of publication of the patent specification: **28.11.90**

(51) Int. Cl.⁵: **C08K 5/34**, C08L 67/02, C07D 209/48

(21) Application number: **86870107.9**

(22) Date of filing: **31.07.86**

(54) **Polyester compositions containing phthalimidoesters.**

(30) Priority: **01.08.85 US 761253**
**02.05.86 US 856384**

(43) Date of publication of application:
**11.03.87 Bulletin 87/11**

(45) Publication of the grant of the patent:
**28.11.90 Bulletin 90/48**

(84) Designated Contracting States:
**AT BE CH DE FR GB IT LI LU NL SE**

(56) References cited:
**DE-C- 855 259**
**FR-A- 2 000 301**
**FR-A- 2 008 608**
**US-A- 3 210 313**
**US-A- 3 579 363**
**US-A- 3 579 364**

**Encyclopedia of Polymer sciences and Engineering, vol. 12, p. 219, 228 (J. Wiley)**

(73) Proprietor: **Monsanto Company, Patent Department 800 North Lindbergh Boulevard, St. Louis Missouri 63167-7020(US)**

(72) Inventor: **Birum, Gail Hubert, 1015 Edgeworth Drive, Kirkwood Missouri 63122(US)**
Inventor: **Jansen, Richard Francis, 2663 Nahn Drive, St. Louis Missouri 63129(US)**
Inventor: **Mathis, Thomas Coleman, 9354 Hazelridge Drive, St. Louis, Missouri 63126(US)**

(74) Representative: **Ernst, Hubert et al, Monsanto Services International S.A. Avenue de Tervuren 270/272, B-1150 Brussels(BE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

## Description

Background of the Invention

This invention relates to polyester compositions. Particularly, the invention relates to polyethylene terephthalate compositions containing certain phthalimidoesters, as plasticizers, and more particularly to compositions for molding engineering thermoplastic parts.

Polyalkylene terephthalates have long been used for the manufacture of various molded articles. Such polymers, particularly when combined with reinforcing materials and fillers such as glass fibers, are suitable for the manufacture of engineered thermoplastic molded articles due to their high wear resistance, durability and high dimensional accuracy. These good physical properties are most readily achieved by molding a composition containing reinforcing material, under conditions under which the polyalkylene terephthalates become partially crystalline.

Of the polyalkylene terephthalates, polyethylene terephthalate imparts preferred physical properties in the molded article. However, polyethylene terephthalate is often not the material of choice for injection molding usage because relatively high mold temperatures, e.g., 120°C to 140°C, must be utilized to ensure good moldability and to obtain the desired crystallinity. Because of the high temperatures required, relatively long molding times are necessary. These stringent processing conditions often prevent the use of polyethylene terephthalate for injection molding in spite of its high rigidity and good heat distortion temperature. Other polyester polymers, particularly polybutylene terephthalate require shorter molding times and lower molding temperatures because of their higher inherent rate of crystallization. However, these polymers are inferior to polyethylene terephthalate in their physical properties, particularly in their heat distortion temperature.

Thus, it is desirable to produce a polyethylene terephthalate molding composition that will crystallize at lower temperatures. This lowering of crystallization temperature must be accomplished without unduly adversely affecting the physical properties of the molded article. Additionally, any additive that improves crystallization must have sufficiently low volatility so that it does not volatilize out of the composition at elevated processing temperatures, and so that it does not cause deposits on the mold surfaces. The phthalimidoesters of the invention reduce the molding time and temperatures of polyethylene terephthalate compositions by improving and reducing the lower temperature at which crystallization occurs during cooling of the melt. Furthermore, such phthalimidoesters also result in polyethylene terephthalate compositions with low volatility, good physical properties, and high gloss.

Summary of the Invention

The present invention provides a polyester composition comprising:

(a) a polyethylene terephthalate having an intrinsic viscosity of at least 0.4 deciliter/gram measured as a 0.5% by weight solution in a 60:40 mixture of phenol and tetrachloroethane at 25°C; and

(b) a plasticizer comprising a phthalimidoester of the formula:

$$X-A-\overset{\overset{O}{\|}}{C}-O-R$$

where X is phthalimido, dihydrophthalimido, tetrahydrophthalimido, or hexahydrophthalimido and where A is alkylene, or substituted alkylene with from 1 to 18 carbon atoms and where R is alkyl, alkenyl, substituted alkyl, or substituted alkenyl with from 4 to 20 carbon atoms, or

$$-R'-O-\overset{\overset{O}{\|}}{C}-A'-X'$$

where X′ may be the same as or different than X and is phthalimido, dihydrophthalimido, tetrahydrophthalimido or hexahydrophthalimido and where R′ is alkylene, alkenylene, substituted alkylene, or substituted alkenylene with from 2 to 20 carbon atoms and A′ is alkylene, or substituted alkylene with from 1 to 18 carbon atoms.

If the phthalimidoester is a monophthalimidoester, it is preferred that the number of carbon atoms in A and R total from 5 to 30 carbon atoms. And if the phthalimidoester is a diphthalimidoester, it is preferred that the carbon atoms of A, R′ and A′ total from 4 to 36 carbon atoms. Preferably A, A′ and R′ are each alkylene and R is alkyl.

It is preferred that the phthalimidoester be at least 0.1% by weight of the polyethylene terephthalate.

In a preferred embodiment, the present invention comprises a polyester molding composition comprising:

(a) from 40 to 95 parts by weight of a polyethylene terephthalate having an intrinsic viscosity of at least 0.4 deciliter/gram measured as a 0.5% by weight solution in a 60:40 mixture of phenol and tetrachloroethane at 25°C;

(b) from 0.1 to 15 parts by weight of a plasticizer comprising a phthalimidoester as defined above;

(c) from 0.1 to 10.0 parts by weight of a nucleating agent; and

(d) from 5 to 60 parts by weight of a reinforcing agent.

This invention also provides a molded, shaped article prepared from the polyester molding composition described above.

This invention also provides a process for the preparation of polyester compositions wherein a polyethylene terephthalate having an intrinsic viscosity of at least 0.4, preferably at least 0.6 deciliters per gram is mixed with phthalimidoester as defined above, and homogenized in a melt.

## Detailed Description of the Invention

The polyethylene terephthalate used in preparing the polyester compositions of the invention has an intrinsic viscosity of at least 0.4 deciliters per gram when measured as a 0.5% by weight solution in a 60:40 mixture of phenol and tetrachloroethane at 25°C. It is preferred that the intrinsic viscosity should be about 0.6 deciliters per gram and in some instances an intrinsic viscosity of about 1.0 or more deciliters per gram may be desirable. As used herein, the term "polyethylene terephthalate" includes polyethylene terephthalate as well as polyethylene terephthalate copolymers and polymer blends containing polyethylene terephthalate, provided the copolymer or polymer blend is at least 60% by weight polyethylene terephthalate. Preferred copolymers include those in which a portion of the ethylene glycol is replaced by other polyhydric alcohols, including, but not limited to, propylene glycol and butylene glycol, and in which a portion of the terephthalic acid is replaced by other polyfunctional carboxylic acids. Suitable polymer blends include physical mixtures of polyethylene terephthalate with other polymers, particularly other polyesters. The polyethylene terephthalate is used in the molding composition in an amount of from 50 to 95 parts by weight, preferably 60 to 90 parts by weight.

The phthalimidoester of this invention comprise compounds of the general formula:

$$X-A-\overset{O}{\overset{\|}{C}}-O-R$$

where X is phthalimido, dihydrophthalimido, tetrahydrophthalimido, or hexahydrophthalimido, and where A is alkylene or substituted alkylene, with from 1 to 18 carbon atoms, preferably from 1 to 12 carbon atoms, and more preferably, from 1 to 6 carbon atoms, and where R is alkyl, substituted alkyl, alkenyl, or substituted alkenyl with from 4 to 20 carbon atoms preferably from 7 to 18 carbon atoms or

$$R'-O-\overset{O}{\overset{\|}{C}}-A'-X'$$

where X′ may be the same as or different than X and is phthalimido, dihydrophthalimido, tetrahydrophthalimido or hexahydrophthalimido and where R′ is alkylene, alkenylene, substituted alkylene, or substituted alkenylene with from 2 to 20 carbon atoms, preferably from 2 to 10 carbon atoms and A′ is alkylene or substituted alkylene with from 1 to 18 carbon atoms, preferably from 1 to 12 carbon atoms, and more preferably, from 1 to 6 carbon atoms.

If the phthalimidoester is a monophthalimidoester, it is preferred that the carbon atoms of A and R total from 5 to 30 carbon atoms, more preferably from 8 to 28 carbon atoms. If the phthalimidoester is a diphthalimidoester, it is preferred that the carbon atoms of A, R′, and A′ total from 4 to 36 carbon atoms, more preferably from 6 to 28 carbon atoms.

In general, the phthalimidoester is present at least 0.1% by weight of the polyethylene terephthalate. In a complete molding composition the amount of phthalimidoester ranges from 0.1 to 15 parts by weight, for example, from 0.5 to 15 parts by weight, and preferably, from 3 to 10 parts by weight. These phthalimidoester compounds have excellent compatibility with polyethylene terephthalate. However, the concentration of phthalimidoester should be kept below a level at which incompatibility may occur under the conditions to which the composition will be exposed.

The phthalimidoesters used in this invention can be prepared by any method known in the art. For example, U.S. Patent No. 3 579 363 describes preparation of 2-ethylhexyl phthalimidoacetate by reacting potassium phthalimide and 2-ethylhexyl α-chloroacetate. It is preferred that the phthalimidoesters be

prepared by reacting phthalic anhydride, dihydrophthalic anhydride, tetrahydrophthalic anhydride, hexahydrophthalic anhydride, or mixtures thereof with either an amino acid or a lactam to form the precursor phthalimidocarboxylic acid which is further reacted with an alcohol to form the phthalimidoester. For sake of simplicity, preparation of the phthalimidoester will be discussed in terms of phthalic anhydride starting material, but the discussion also applies to the hydrogenated phthalic anhydrides.

When an amino acid is used, the phthalimidoester may be prepared in a two-step reaction. In the first step, the amino acid is reacted with phthalic anhydride, usually in the presence of a solvent, such as toluene, xylene, or diethylbenzene, to produce the precursor phthalimidocarboxylic acid. In the second step, this intermediate is esterified by reaction with an appropriate alcohol, preferably in the presence of an esterification catalyst, such as stannous oxalate or other esterification catalyst, and preferably in the presence of an entrainer, which removes water released by the esterification reaction, either by reacting with the water or by forming an azeotrope to remove the water from the reaction mixture. A suitable entrainer is diethyl benzene.

The phthalimidoester may also be prepared in a single step by mixing phthalic anhydride, the amino acid, the alcohol, a catalyst, if desired, and a solvent, if desired, and maintaining the reaction mixture at reflux to remove the water as it evolves. Excess alcohol can be used as the solvent and as an entrainer. After the reaction is complete, unreacted alcohol can be removed by steam stripping, which also insolubilizes the stannous oxalate catalyst for easy removal.

Similarly, either a one-step or two-step process can be used with lactams. The one step process is preferred with either amino acids or lactams, because of simplicity, and the ability to avoid introducing a solvent to the reaction mixture. Additionally, if a lactam is used, the reaction proceeds at a higher rate if a one-step process is used.

Any amino acid can be used as a starting material, provided it does not contain substituents that interfere with formation of the phthalimidoester, that render the resulting phthalimidoester incompatible with the polyethylene terephthalate, or that render it ineffective. Examples of suitable amino acids include glycine, α-alanine, β-alanine, aminodecanoic acid, methionine, 3-aminobutyric acid, 6-aminocaproic acid, isoleucine, 2-amino-4-methylpentanoic acid, 2-amino-3-phenylpropionic acid, 2-amino-3-methylbutyric acid, aminooctadecanoic acid, or any other amino acid with a suitable alkylene or substituted alkylene group. A mixture of suitable amino acids can also be used.

Any lactam can be used as a starting material, provided it does not contain susbtituents that interfere with formation of the phthalimidoester or that render the resulting phthalimidoester incompatible with the polyethylene terephthalate or that render it ineffective. Examples of suitable lactams include 2-pyrrolidone, δ-valerolactam, ε-caprolactam, or any other lactam with asuitable alkylene or substituted alkylene group. Mixtures of suitable lactams or of lactams and amino acids can also be used.

When referring to amino acids, lactams, and groups A and A', the phrase alkylene or substituted alkylene is meant to include both straight and branched chain groups as well as groups that contain substituents that do not interfere with formation of the phthalimidoester, and that do not render the resulting phthalimidoester incompatible with the polyetheylene terephthalate or ineffective. Examples of noninterfering substituents include ether and thioether groups.

If monohydric alcohols are used for esterification, monophthalimide compounds result. If dihydric alcohols are used, diphthalimide compounds result. Any monohydric alcohol can be used, provided it has from 4 to 20 carbon atoms, preferably 7 to 18 carbon atoms, and does not contain substituents that interfere with formation of the phthalimidoester or that render the resulting phthalimidoester incompatible with the polyethylene terephthalate or ineffective. Examples of suitable monohydric alcohols include heptanol, octanol, nonanol, decanol, undecanol, dodecanol, octadecanol, or any other suitable monohydric alcohol or mixture of monohydric alcohols. Any dihydric alcohol can be used provided it has from 2 to 20 carbon atoms, preferably from 2 to 10 carbon atoms, and does not contain susbtituents that interfere with formation of the phthalimidoester or that render the resulting phthalimidoester incompatible with the polyethylene terephthalate or ineffective. Examples of suitable dihydric alcohols include ethylene glycol, propylene glycol, neopentyl glycol, 1,4-butylene glycol, 1,6-hexanediol, 1,12-dodecanediol, 2-butene-1,4-diol, diethylene glycol, triethylene glycol, or other suitable glycols or glycol oligomers or mixtures of dihydric alcohols. When referring to the alcohols, or to groups R and R', the phrases alkyl, alkenyl, alkylene, substituted alkylene, alkenylene, and substituted alkenylene are meant to include both straight and branched chain groups, as well as groups that contain other substituents that do not interfere with formation of the phthalimidoester, or render the resulting phthalimidoester either incompatible with the polyethylene terephthalate or inactive.

Mixtures of monohydric or dihydric alcohols can also be used, which will result in a product that is a mixture of monophthalimidoester and diphthalimidoester.

Any nucleating agents suitable for use in polyalkylene terephthalate molding compositions may be utilized in the molding compositions of the invention. All that is required is that the nucleating agent promotes nucleation of the polyalkylene terephthalate crystals. Most nucleating agents raise the upper temperature at which crystallization of the polyethylene terephthalate occurs as the melt cools. Suitable nucleating agents include organic and inorganic nucleators.

Inorganic nucleating agents include calcium salts, such as calcium terephthalate, calcium titanate, calcium pyrophosphate, calcium silicate, calcium benzoate, calcium oxide, calcium carbonate, calcium fluoride and calcium aluminosilicate. Sodium salts, such as sodium silicate, sodium phenyl phosphate, and sodium aluminosilicate may be used. Other inorganic nucleating agents include salts of other metals, such as lithium, potassium, rubidium and cesium, as well as other inorganic compounds such as zinc borate and zeolites.

One suitable class of inorganic nucleating agents include monovalent metal salts of oxides of carbon, silicon, germanium, tin, and lead. The preferred oxides are those of carbon and silicon, with carbon oxides such as carbonate and bicarbonate being particularly preferred. Preferred monovalent metals include lithium, sodium, potassium, rubidium, and cesium, with lithium, sodium and potassium being preferred, and sodium being particularly preferred.

Other suitable nucleating agents include the sodium or potassium, or other metal salts of hydrocarbon carboxylic acids. One class of suitable carboxylic acids contains between 7 and 25 carbon atoms, preferably more than 12 carbon atoms. Representative of these carboxylic acids are fatty acids, such as stearic, pelargonic and behenic acid. Additionally, salts of ethylenediaminetetraacetic acid may be suitable nucleating agents, particularly the sodium salt. These carboxylic acid salts also include the sodium or potassium salts of carboxyl containing organic polymers, either fully or partially neutralized, such as copolymers of olefins or aromatic olefins with acrylic or methacrylic acids or maleic anhydride. These polymeric materials include, for example, the sodium or potassium salt of ethylene methacrylic acid copolymers or styrene maleic anhydride copolymers, including both wholly or partially neutralized salts of each. In the copolymers above, the olefin or aromatic olefin moiety ordinarily comprises 50% to 98% by weight of the copolymer and, preferably, 80% to 98%. An especially preferred polymeric material is the sodium salt of ethylene methacrylic acid copolymer. Also included are salts or oligomers of unsaturated fatty acids, particularly dimers and trimers of $C_{18}$ fatty acids, commonly known as "dimer acid" and "trimer acid" and mixtures thereof.

A preferred class of nucleators, whether organic or inorganic, is alkali metal salts and mixed salts containing alkali metals. Preferred alkali metals are sodium and potassium, with sodium being particularly preferred.

A suitable amount of nucleating agent in 100 parts by weight of molding composition is often 0.5 to 5.0 parts by weight.

In general, any suitable reinforcing agent can be used in the molding compositions of the invention. The reinforcing agent may optionally be treated with various coupling agents or adhesion promoters in a manner in which is well known to those skilled in the art. Examples of suitable reinforcing agents include glass fibers, carbon fibers and filaments, aramid fibers, alumina, feldspar, asbestos, talc, calcium carbonates, clay, carbon black, quartz, novaculite and other forms of silica, galenite, bentonite, garnet, mica, saponite, beidellite, titanium dioxide and titanate whiskers, aluminum, iron or nickel fibers, whiskers or platelets, vermiculite and calcium metasilicate. In particular, the preferred reinforcing agent is glass fiber, more particularly glass fibers, comprised of lime-aluminum borosilicate glass that is relatively sodium free, commonly known as "E" glass. In some instances, it also may be desirable to use a mixture of reinforcing agents such as a mixture of glass fibers and mica.

Other optional additives may be included in the compositions of the invention. For example, the compositions of the invention may also include a chain extender which helps to compensate for polyester chains which are broken by hydrolysis with resulting molecular weight degradation. Such chain extenders include, for example, carbodiimides and polyepoxides. Epoxy resins, which are preferred, include an epoxy formed from bisphenol-A and glycidol ether or polyepoxides obtained by reacting ortho cresol novolac and epichlorohydrin. Especially preferred polyepoxides are epoxy cresol novolac resins. If a chain extender is used, it is preferred that 0.1 to 5 parts by weight be used. Other optional additives may include impact modifiers, flow bromoters, mold release agents, anti-static agents, coloring agents, such as pigments and dyes, thermal oxidative and light stabilizers, flame retardants coupling agents and other additives known in the art.

The molding compositions of the invention can be prepared and molded using any conventional or well-known method. For example, in one suitable method the polyalkylene terephthalate and glass fiber are placed into an extrusion compounder to produce molding pellets. In another procedure, the polyalkylene terephthalate and glass are mixed by dry blending, then either milled and comminuted or extruded and chopped. Alternatively, the ingredients can be mixed with the powdered or granular polyalkylene resin and directly molded by injection or transfer molding techniques. Ordinarily, it is desirable to thoroughly free the ingredients from as much water as possible.

It is preferred that compounding be carried out to ensure that the residence time in the molding machine is short; the temperature is carefully controlled; friction heat is utilized; and an intimate blend between the additives and the polyester resin is obtained.

Although it is not essential, best results are obtained if the ingredients are precompounded, pelletized and then molded. Precompounding can be carried out in conventional equipment. For example, after predrying the polyalkylene terephthalate, e.g., at 130°C for three hours, a single screw extruder may be fed with a dry blend of the polyester resin and the glass and whatever other additives may be used. Alternatively, a twin-screw extrusion machine can be fed with polyalkylene terephthalate, glass fiber and other additives at the feed port. In either case, a generally suitable extruder temperature will be 230°C to 300°C.

The molding compositions of this invention can be molded in any equipment conventionally used for molding engineering thermoplastic compositions, using conventional techniques.

The phthalimidoester compounds of this invention act to improve the crystallization of the polyethylene terephthalates to which they are added by decreasing the temperature to which crystallization will continue upon cooling of the hot melt. Although not intending to be bound by theory, it is believed that the phthalimidoesters improve crystallization by increasing the molecular mobility of the polymer chain.

Additionally, the phthalimidoesters of this invention result in improved melt flow and in improved processability of polyethylene terephthalate compositions, intended for molding or other applications. As a result, the phthalimidoesters can also be considered to be a flow aid or processing aid for polyethylene terephthalate.

Because the phthalimidoesters of this invention decrease the temperature at which crystallization occurs, good heat distortion temperatures (HDT), gloss and other physical properties can be obtained at a lower molding temperature. Also, smaller amounts of nucleating agents may be used without any loss of nucleating effectiveness.

The compositions of this invention can be prepared using compounding techniques known in the art. They may either be precompounded and pelletized, or mixed and compounded as part of the molding process. It is preferred to precompound and pelletize the composition prior to molding. Precompounding can be accomplished with a single screw extruder, dual screw extruder, a roller mill, or any other method known to one skilled in the art.

The following examples are illustrative of this invention and are not intended to limit its scope.

In the molding formulations of the examples, the following components were used: the polyethylene terephthalate (PET) used had an intrinsic viscosity of about 0.66 deciliters/gram; the indicated plasticizer; as a nucleator, an ethylene/methacrylic acid (85/15 by weight) partially neutralized to a sodium salt, sold by duPont under the tradename Surlyn 8660; as a chain extender, a polyglycidyl ether of orthocresolformaldehyde novolac, sold by Ciba-Geigy Corporation, under the tradename Araldite ECN 1273; and 1/8-inch (0.3175 cm) chopped glass strand, sold by Pittsburgh Plate Glass and designated as product type 3540. Compositions were made using plasticizer at a concentration of 5% by weight based on PET. This formulation, expressed as a percentage of the full formulation is as follows:

| | % Plasticizer Based on PET |
|---|---|
| | 5% |
| PET | 65.6 |
| Plasticizer | 3.4 |
| Nucleator | 0.67 |
| Chain Extender | 0.33 |
| Glass | 30.0 |

The dried components were mixed and extruded in a single screw extruder, with a die temperature of about 260°C. The extruded ribbon was cooled, chopped into pellets and dried overnight. The dried, pelletized composition was injection molded into a family mold, at a die temperature of about 275°C and a mold surface temperature of 110°C. The closed time was 30 seconds.

The molded parts were tested to determine heat deflection temperature (HDT) by holding the specimen at two support points separated by five inches (12.7 cm), raising the temperature 2°C per minute, and applying a load of 264 psi (1822 kPa) at the midpoint. The temperature at which deformation of the specimen reaches 0.01 inches (.0254 cm) is the HDT. This is in accordance with ASTM D648-82. Tensile strength and % elongation were determined using ASTM D638-82. Izod impact was determined using ASTM D256-81, except that untouched test specimens were used. Volatility of the compound PET was determined by heating for 4 hours at 175°C on a rotating rack in a forced draft oven.

The $T_{pk}$ of the composition was determined by placing a sample of a molded part into the sample container of a differential scanning calorimeter, that had been heated to about 290°C. After 2 minutes, the sample container was removed and covered with powdered dry ice to "quench" the sample. The quenched sample was dessicated a minimum of 5 minutes, then returned to the calorimeter that had been cooled to room temperature. The calorimeter was programmed for a 10°C per minute temperature increase under a nitrogen atmosphere. An exotherm was observed between about 100°C and about 125°C. The $T_{pk}$ is the temperature at which heat evolves most rapidly during this exotherm. The $T_{pk}$ gives an indication of the effectiveness of the plasticizer at enhancing crystallization with a lower $T_{pk}$ indicating greater effectiveness.

Additionally, the per se volatility of the indicated plasticizer was determined by thermogravimetric analysis of the neat plasticizer, under air atmosphere, using a thermobalance with a programmed temperature rise of 10°C per minute. The thermobalance produced a plot of weight loss versus temperature.

From this plot, the temperature at which volatility losses began was determined, as well as the weight percent loss at 300°C and 350°C.

EXAMPLE 1

This example illustrates the stepwise preparation of undecyl 3-phthalimidoproprionate by the initial preparation and isolation of the precursor acid 3-phthalimidopropionic acid, followed by esterification with undecyl alcohol.

A 500 ml flask, equipped with a stirrer, thermometer, and water-separating condenser, was charged with 0.5 mole of β-alanine and 170 ml of xylene. This mixture was stirred and the temperature maintained at 110°-120°C as 0.5 moles of phthalic anhydride was added in portions over 50 minutes. Warming was continued at reflux until water was no longer collected. The reaction mixture was then cooled to room temperature, filtered and the solid washed with xylene and dried. The product was 3-phthalimidopropionic acid, with a melting point of 138°-146°C.

A 0.44 mole portion of the precursor acid, 0.5 moles of undecyl alcohol, 0.3g of stannous oxalate catalyst, and 50 g of diethylbenzene entrainer were charged to the equipment described above. This mixture was warmed at 210°C, with pressure reduced to maintain reflux, until water was no longer collected. The reaction mixture was then steam-stripped at 165°C and 30 mm Hg, and the product was filtered, giving a 94.5% overall yield of undecyl 3-phthalimidopropionate.

EXAMPLE 2

This example describes the one step production of undecyl 3-phthalimidopropionate in which all the reactants were charged initially.

A 1-liter flask, equipped as in Example 1 was charged with 1.0 mole of phthalic anhydride, 1.0 moles of β-alanine, 1.15 moles of undecyl alcohol, and 0.7g of stannous oxalate. The mixture was warmed to 210°C and kept at this temperature, with the pressure reduced to maintain reflux, until water was no longer collected. The reaction mixture was then steam-stripped as in Example 1, and the product was filtered, giving a 99.6% yield of undecyl 3-phthalimidopropionate.

EXAMPLE 3

This example describes the stepwise preparation of undecyl 6-phthalimidohexanoate by the initial preparation and isolation of the precursor acid, 6-phthalimidohexanoic acid, followed by esterification with undecyl alcohol.

A 1-liter flask equipped as in Example 1 was charged with 0.76 moles of 6-aminohexanoic acid and 250 ml of xylene. This mixture was stirred and the temperature was maintained at 115°C-120°C as 0.76 moles of phthalic anhydride was added in portions over 25 minutes. The reaction mixture was warmed at reflux until water was no longer collected. More xylene was added to aid stirring when the mixture cooled to room temperature. The reaction mixture was filtered, and the solid was washed with xylene and dried to give an 84.5% yield of 6-phthalimidohexanoic acid with a melting point of 105°-110°C.

A 0.59 mole portion of the precursor acid, 0.6 moles of undecyl alcohol, 0.5g of stannous oxalate catalyst, and 50 g of diethylbenzene entrainer were charged to the equipment described above. This mixture was warmed at 210°C, with the pressure reduced to maintain reflux, until water was no longer collected. The reaction mixture was then steam-stripped as in Example 1 and the product was filtered to isolate the undecyl 6-phthalimidohexanoate.

EXAMPLE 4

This example describes the use of ε-caprolactam and the simultaneous charging of all three reactants to produce undecyl 6-phthalimidohexanoate, without isolation of the intermediate acid.

The equipment used in Example 1 was charged with 0.89 moles of ε-caprolactam, 0.89 moles of phthalic anhydride, 0.92 moles of undecyl alcohol, and 60g of diethylbenzene entrainer. This mixture was warmed at 175°C for 1 hour and then 0.7g of stannous oxalate catalyst was added and warming was continued at 210°C for 3 hours with the pressure reduced to maintain reflux. 10g more of undecyl alcohol was added, and refluxing was continued. The reaction mixture was steam-stripped as above and filtered, resulting in a mixture of approximately 95% undecyl 6-phthalimidohexanoate and 5% other components.

EXAMPLE 5

Octadecyl 3-phthalimidopropionate was prepared by a procedure similar to that used in Example 1, except that the intermediate acid was not isolated before it was esterified with octadecyl alcohol. The product had a proton NMR spectrum that was consistent with the desired structure.

EXAMPLE 6

Octadecyl 6-phthalimidohexanoate was prepared from phthalic anhydride, ε-caprolactam, and octade-

cyl alcohol by a procedure similar to that used in Example 4. The product has a proton NMR spectrum consistent with the desired structure.

EXAMPLE 7

Octadecyl 11-phthalimidoundecanoate was prepared from phthalic anhydride, 11-aminoundecanoic acid, and undecyl alcohol by a procedure similar to that used in Example 1, except that the intermediate acid was not isolated before it was esterified. The product had a proton NMR spectrum that was consistent with the desired structure.

EXAMPLE 8

Undecyl 2-phthalimido-4-(methylthio)butyrate was prepared from phthalic anhydride, DL-methionine, and undecyl alcohol by a procedure similar to that used in Example 1, except that the intermediate acid was not isolated before it was esterified. The product had a proton NMR spectrum consistent with the desired structure.

EXAMPLE 9

Octadecyl 2-phthalimidoacetate was prepared from phthalic anhydride, glycine and octadecyl alcohol by a procedure similar to that used in Example 1, except that the intermediate acid was not isolated from its reaction medium before it was esterified. The product had a proton NMR spectrum consistent with the desired structure.

EXAMPLE 10

A mixture of octadecyl and hexadecyl 4-phthalimidobutyrate was prepared from phthalic anhydride 4-aminobutyric acid, and tallow-derived $C_{16-18}$ alcohol by a procedure similar to that used in Example 1 except that the intermediate acid was not isolated from the reaction mixture before it was esterified. The product had a proton NMR spectrum consistent with the desired structure.

EXAMPLE 11

Undecyl 2-phthalimidopropionate was prepared from phthalic anhydride, DL-alanine, and undecyl alcohol by a procedure similar to that used in Example 1, except that the intermediate acid was not isolated from its reaction medium before it was esterfied. The resultant product had a proton NMR spectrum consistent with the desired structure.

EXAMPLE 12

A mixture of heptyl, nonyl, and undecyl 6-phthalimidohexanoates was prepared from phthalic anhydride, ε-caprolactam, and a mixture of about 30% $C_7$ alcohol, about 40% $C_9$ alcohol, and about 30% $C_{11}$ alcohol, by a procedure similar to that used in Example 4. The product had a proton NMR spectrum consistent with the desired structure.

EXAMPLE 13

2-Ethylhexyl 6-phthalimidohexanoate was prepared from phthalic anhydride, ε-caprolactam and 2-ethylhexyl alcohol by a procedure similar to that used in Example 4. The product had a proton NMR spectrum consistent with the desired structure.

EXAMPLE 14

Dodecyl 6-phthalimidohexanoate was prepared from phthalic anhydride, ε-caprolactam, and dodecyl alcohol by a procedure similar to that used in Example 4. The product had a proton NMR spectrum consistent with the desired structure.

EXAMPLE 15

Undecyl 2-phthalimidoacetate was prepared by adding 2.2 moles of undecyl alcohol to a suspension of 2 moles of 2-phthalimidoacetic acid in xylene. The mixture was stirred and warmed, allowing xylene and the water of reaction to distill until a pot temperature of 210°C was reached at a pressure of about 250 mm Hg. Refluxing at this temperature was continued until water no longer was produced. The reaction mixture was steam-stripped as above and filtered yielding a product having a proton NMR spectrum consistent with the desired structure.

EXAMPLE 16

The di(3-phthalimidopropionate) diester of 2,2-dimethyl-1,3-propanediol was prepared with phthalic anhydride, β-alanine and the dihydric alcohol by a procedure similar to that used in Example 1. The product had a melting point of 158-162°C, and its proton NMR spectrum was consistent with the desired structure.

EXAMPLE 17

The di(6-phthalimidohexanoate) diester of 2,2-dimethyl-1,3-propanediol was prepared from phthalic anhydride, 6-aminohexanoic acid, and the dihydric alcohol by a procedure similar to that used in Example 1 except that the intermediate acid was not isolated from its reaction medium before it was esterified. The product had a proton NMR spectrum that was consistent with the desired structure.

EXAMPLE 18

The di(6-phthalimidohexanoate) diester of triethyleneglycol was prepared from phthalic anhydride, β-caprolactam, and triethyleneglycol by a procedure similar to that used in Example 4. The product was a clear amber liquid having a proton NMR spectrum that was consistent with the desired structure.

EXAMPLE 19

Undecyl 6-tetrahydrophthalimidohexanoate was prepared from 1.2 moles of 1,2,3,6-tetrahydrophthalic anhydride, 1.2 moles of ε-caprolactam, and 1.56 of undecanol in a 1-liter flask equipped as in Example 1. The mixture was warmed at 180°C under nitrogen for 1¼ hours and 0.7g of stannous oxalate catalyst was added. The mixture was heated to 250°C for 3¼ hours with the pressure reduced to maintain reflux. About 21 ml of water was collected. The reaction mixture was then steam stripped at 175°C and 10 mm Hg for 2 hours, followed by continued stripping without steam for ¼ hour. The mixture was filtered giving a clear yellow liquid product. The product had a proton NMR that was consistent with the desired structure.

The products of the synthesis examples above were formulated into polyethylene terephthalate molding compositions and were molded as described above. Various of the phthalimidoesters were tested in groups, some of which were compared with similar compositions made using a mixture of $C_{14}$, $C_{16}$, and $C_{18}$ N-alkyl, toluenesulfonamide (TSA), and some were compared with similar formulations using no plasticizer. The properties of the plasticizers and of the formulated PET are reported in Tables I through V below.

TABLE I

| Ex. No. | Mol. Wt. | Melting Point (°C) | Plasticizer Volatility: Loss Begins (°C) | % Loss 300°C | % Loss 350°C | $T_{pk}$(°C) | HDT(°C) | Izod† Impact (ft.lbs/in) | Tensile Properties: Strength (psi)‡ | % Elong. | PET Form. at 175°C (mg/sq.cm) |
|---|---|---|---|---|---|---|---|---|---|---|---|
| 1 | 374 | 50-57 | 215 | 18 | 49 | 107.5 | 214 | 13.4 | 19,250 | 2.0 | 0.82 |
| 3 | 416 | RT* | 200 | 56 | 88 | 106.5 | 210 | 12.3 | 19,250 | 2.0 | 0.72 |
| 5 | 472 | 73-79 | 225 | 13 | 45 | 107.0 | 219 | 13.5 | 19,250 | 2.0 | 0.41 |
| 7 | 485 | 43-48 | 240 | 10 | 31 | 106.0 | 210 | 12.5 | 19,500 | 2.0 | 0.34 |
| TSA | 418 | 68-70 | 265 | 2 | 16 | 107 | 218 | 13.3 | 19,500 | 2.0 | 0.52 |

*Room temperature

† to convert ft.lbs/in to J/m Multiply by 53·38

‡ to convert psi to kPa Multiply by 6·89

TABLE II

| Ex. No. | Mol. Wt. | Melting Point (°C) | Plasticizer Volatility: Loss Begins (°C) | Plasticizer Volatility: % Loss 300°C | Plasticizer Volatility: % Loss 350°C | $T_{pk}$(°C) | HDT(°C) | Izod Impact (ft.lbs/in) | Tensile Properties: Strength (psi) | Tensile Properties: % Elong. | PET Form. at 175°C (mg/sq.cm) |
|---|---|---|---|---|---|---|---|---|---|---|---|
| 17 | 591 | Liquid* | 290 | 1 | 6 | 110.5 | 207 | 12.6 | 19,950 | 2.0 | 0.27 |
| 6 | 514 | 47 | 190 | 35 | 73 | 107.0 | 218 | 12.7 | 19,100 | 2.0 | 0.36 |
| 16 | 507 | 158-162 | 310 | 0 | 5 | 112.0 | 206 | 12.2 | 19,600 | 1.9 | 0.23 |
| None | | | - | - | - | 120.0 | 207 | 12.2 | 18,350 | 1.9 | 0.07 |
| TSA | 418 | 68-70 | 265 | 2 | 16 | 107.0 | 211 | 12.2 | 19,450 | 2.0 | 0.48 |

*Room temperature

TABLE III

| Ex. No. | Mol. Wt. | Melting Point (°C) | Plasticizer Volatility: Loss Begins (°C) | Plasticizer Volatility: % Loss 300°C | Plasticizer Volatility: % Loss 350°C | $T_{pk}$(°C) | HDT(°C) | Izod Impact (ft.lbs/in) | Tensile Properties: Strength (psi) | Tensile Properties: % Elong. | PET Form. at 175°C (mg/sq.cm) |
|---|---|---|---|---|---|---|---|---|---|---|---|
| 8 | 434 | Liquid* | 225 | 9 | 43 | 108.0 | 218 | 10.7 | 19,500 | 1.9 | 0.46 |
| 9 | 458 | 70-75 | 235 | 8 | 30 | 108.0 | 216 | 11.4 | 20,350 | 2.0 | 0.45 |
| 11 | 374 | Liquid* | 185 | 76 | 96 | 108.5 | 221 | 11.1 | 20,100 | 2.0 | 0.66 |
| 12 | 385 | Liquid* | 195 | 74 | 93 | 105.0 | 220 | 12.1 | 19,500 | 2.0 | 0.80 |
| 13 | 374 | Liquid* | 190 | 73 | 93 | 106.5 | 214 | 12.0 | 18,400 | 1.6 | 0.71 |
| None | | | - | - | - | 119.0 | 212 | 14.0 | 19,000 | 1.9 | 0.10 |
| TSA | 418 | 68-70 | 265 | 2 | 16 | 107.0 | 216 | 11.6 | 20,300 | 2.0 | 0.48 |

*Room temperature

TABLE IV

| Ex. No. | Mol. Wt. | Melting Point (°C) | $T_{pk}$-(°C) | HDT(°C) | Izod Impact (ft.lbs/in) | Tensile Properties Strength (psi) | Tensile Properties % Elong. | PET Form. at 175°C (mg/sq.cm) |
|---|---|---|---|---|---|---|---|---|
| 14 | 430 | 36-38 | 106.0 | 214 | 11.7 | 19,450 | 2.0 | 0.63 |
| 15 | 359 | 55-64 | 109.5 | 214 | 12.1 | 19,700 | 2.0 | 0.82 |
| 18 | 637 | Liquid* | 109.5 | 218 | 11.3 | 19,550 | 1.9 | 0.22 |
| None | - | - | 119.5 | 202 | 12.8 | 18,700 | 2.0 | 0.13 |
| TSA | 418 | 68-70 | 107.5 | 214 | 11.7 | 19,400 | 2.1 | 0.54 |

*Room temperature

TABLE V

| Ex. No. | Mol. Wt. | Melting Point (°C) | $T_{pk}$(°C) | HDT(°C) | Izod Impact (ft.lbs/in) | Tensile Properties Strength (psi) | % Elong. | PET Form. at 175°C (mg/sq.cm) |
|---|---|---|---|---|---|---|---|---|
| 19 | 420 | ~RT* | 107.5 | 215 | 12.1 | 19,100 | 2.0 | 0.55 |
| 3 | 416 | ~RT* | 107 | 214 | 11.6 | 19,250 | 2.2 | 0.75 |
| TSA | 418 | 68-70 | 107.5 | 214 | 13.1 | 19,350 | 2.0 | 0.50 |

* Room Temperature

In addition to the compositions discussed above, glass reinforced PET molding compositions were prepared utilizing 2-ethylhexyl 6-tetrahydrophthalimidohexanoate, 2-ethylhexyl 6-hexahydrophthalimidohexanoate and TSA. Parts molded from each of these compositions in a mold with a surface temperature of about 107°C exhibited equivalent physical properties, while the two hydrogenated phthalimidoesters produced surface gloss somewhat superior to the TSA control indicting equivalent or greater crystallization than that resulting from the TSA.

The preceding examples are intended to illustrate the current invention and not to limit its scope.

## Claims

1. A polyester composition comprising:
(a) a polyethylene terephthalate having an intrinsic viscosity of at least 0.4 deciliter/gram measured as a 0.5% by weight solution in a 60:40 mixture of phenol and tetrachloroethane at 25°C; and
(b) a plasticizer comprising a phthalimidoester of the formula:

$$X-A-\overset{\underset{\|}{O}}{C}-O-R$$

where X is phthalimido, dihydrophthalimido, tetrahydrophthalimido or hexahydrophthalimido and where A is alkylene or substituted alkylene with from 1 to 18 carbon atoms and where R is alkyl, substituted alkyl, alkenyl, or substituted alkenyl, with from 4 to 20 carbon atoms, or

$$R'-O-\overset{\underset{\|}{O}}{C}-A'-X'$$

where X′ is the same as or different than X and is phthalimido, dihydrophthalimido, tetrahydrophthalimido or hexahydrophthalimido and where R′ is alkylene, alkenylene, substituted alkylene, or substituted alkenylene with from 2 to 20 carbon atoms and A′ is alkylene or substituted alkylene with from 1 to 18 carbon atoms.

2. The composition of Claim 1, wherein the phthalimidoester comprises at least 0.1% by weight of the polyethylene terephthalate.

3. A polyester molding composition comprising:
(a) from 40 to 95 parts by weight of a polyethylene terephthalate having an intrinsic viscosity of at least 0.4 deciliter/gram measured as a 0.5% by weight solution in a 60:40 mixture of phenol and tetrachloroethane at 25°C;
(b) from 0.1 to 15 parts by weight of a plasticizer comprising a phthalimidoester of the formula:

$$X-A-\overset{\underset{\|}{O}}{C}-O-R$$

where X is phthalimido, dihydrophthalimido, tetrahydrophthalimido, or hexahydrophthalimido, and where A is alkylene or substituted alkylene with from 1 to 18 carbon atoms and where R is alkyl, substituted alkyl, alkenyl, or substituted alkenyl, with from 4 to 20 carbon atoms, or

$$R'-O-\overset{\underset{\|}{O}}{C}-A'-X'$$

where X′ is the same as or different than X and is phthalimido, dihydrophthalimido tetrahydrophthalimido, or hexahydrophthalimido and where R′ is alkylene, substituted alkylene, alkenylene, or substituted alkenylene with from 2 to 20 carbon atoms and A′ is alkylene or substituted alkylene with from 1 to 18 carbon atoms.
(c) from 0.1 to 10.0 parts by weight of a nucleating agent; and
(d) from 5 to 60 parts by weight of a reinforcing agent.

4. The composition of Claim 3, wherein A, A′, and R′ are each alkylene, and R is alkyl.

5. The composition of Claim 3, wherein the phthalimidoester comprises a monophthalimidoester in which the total number of carbon atoms of A and R is from 5 to 30 carbon atoms.

6. The composition of Claim 3, wherein the phthalimidoester comprises a diphthalimidoester in which the total number of carbon atoms of A, R′ and A′ is from 4 to 36 carbon atoms.

7. The composition of Claim 3, wherein the nucleating agent is selected from the salt of a dimer acid, the salt of a trimer acid or the salt of a mixture of a dimer acid and a trimer acid.

8. The composition of Claim 3, wherein the nucleating agent is a copolymer comprising 85 wt.% ethylene and 15 wt.% methacrylic acid which has been partially neutralized with sodium ions.

9. The composition of Claim 3, wherein the reinforcing agent is fiberglass.

10. The composition of Claim 3 containing from 0.1 to 5 parts by weight of a chain extender.

11. The composition of Claim 10, wherein the chain extender is a polyepoxide.

12. The composition of Claim 3, wherein the polyethylene terephthalate has an intrinsic viscosity of at least 0.6.

13. The composition of Claim 3, wherein the polyethylene terephthalate comprises a mixture of polyethylene terephthalate and polybutylene terephthalate.

14. The composition of Claim 3 further comprising at least one additive selected from impact modifiers, flow promoters, coloring agents, flame retardants, coupling agents and stabilizers for thermal oxidative and light stabilization in effective amounts.

15. A polyester molding composition, comprising:

(a) from 40 to 95 parts by weight of polyethylene terephthalate having an intrinsic viscosity of at least 0.4 deciliters per gram;
(b) from 0.5 to 5.0 parts by weight of a nucleating agent;
(c) from 5 to 60 parts by weight of fiberglass;
(d) from 0.5 to 15 parts by weight of a plasticizer comprising a phthalimidoester of the formula:

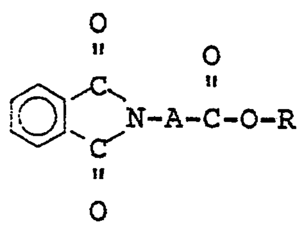

where A is alkylene or with from 1 to 12 carbon atoms and where R is alkyl, or alkenyl with from 4 to 20 carbon atoms, or

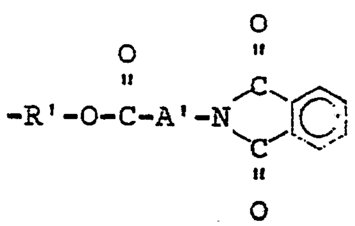

where R′ is alkylene or alkenylene with from 2 to 10 carbon atoms and A′ is alkylene with from 1 to 12 carbon atoms.

16. A polyester molding composition, comprising:

(a) from 40 to 95 parts by weight of polyethylene terephthalate having an intrinsic viscosity of at least 0.4 deciliters per gram;
(b) from 0.5 to 5.0 parts by weight of a nucleating agent;
(c) from 5 to 60 parts by weight of fiberglass;
(d) from 0.5 to 15 parts by weight of a plasticizer comprising a phthalimidoester of the formula:

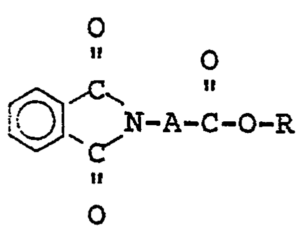

where A is alkylene or substituted alkylene with from 1 to 12 carbon atoms and where R is alkyl, substituted alkyl, alkenyl, or substituted alkenyl with from 4 to 20 carbon atoms.

**Patentansprüche**

1. Polyesterzusammensetzung umfassend
(a) ein Polyäthylenterephthalat mit einer Strukturviskosität von mindestens 0,4 dl/g, gemessen als 0,5 gew.%ige Lösung in einer 60:40 Mischung von Phenol und Tetrachloräthan bei 25°C, und
(b) einen Weichmacher umfassend einen Phthalimidoester der Formel

$$X - A - \overset{O}{\overset{\|}{C}} - O - R \quad ,$$

worin X Phthalimido, Dihydrophthalimido, Tetrahydrophthalimido oder Hexahydrophthalimido bedeutet, A Alkylen oder substituiertes Alkylen mit 1 bis 18 Kohlenstoffatomen darstellt und R Alkyl, substituiertes Alkyl, Alkenyl oder substituiertes Alkenyl mit 4 bis 20 Kohlenstoffatomen ist, oder

$$-R' - O - \overset{O}{\overset{\|}{C}} - A' - X' \quad ,$$

worin X′ gleich X oder davon verschieden ist und Phthalimido, Dihydrophthalimido, Tetrahydrophthalimido oder Hexahydrophthalimido bedeutet, R′ Alkylen, Alkenylen, substituiertes Alkylen oder substituiertes Alkenylen mit 2 bis 20 Kohlenstoffatomen darstellt und A′ Alkylen oder substituiertes Alkylen mit 1 bis 18 Kohlenstoffatomen ist.

2. Zusammensetzung nach Anspruch 1, worin der Phthalimidoester mindestens 0,1 Gew.% des Polyäthylenterephthalats ausmacht.

3. Polyesterformmasse umfassend
(a) 40 bis 95 Gew.Teile eines Polyäthylenterephthalats mit einer Strukturviskosität von mindestens 0,4 dl/g, gemessen als 0,5 gew.%ige Lösung in einer 60:40 Mischung von Phenol und Tetrachloräthan bei 25°C;
(b) 0,1 bis 15 Gew.Teile eines Weichmachers umfassend einen Phthalimidoester der Formel

$$X - A - \overset{O}{\overset{\|}{C}} - O - R \quad ,$$

worin X Phthalimido, Dihydrophthalimido, Tetrahydrophthalimido oder Hexahydrophthalimido bedeutet, A Alkylen oder substituiertes Alkylen mit 1 bis 18 Kohlenstoffatomen darstellt und R Alkyl, substituiertes Alkyl, Alkenyl oder substituiertes Alkenyl mit 4 bis 20 Kohlenstoffatomen ist, oder

$$-R' - O - \overset{O}{\overset{\|}{C}} - A' - X' \quad ,$$

worin X′ gleich X oder davon verschieden ist und Phthalimido, Dihydrophthalimido, Tetrahydrophthalimido oder Hexahydrophthalimido bedeutet, R′ Alkylen, substituiertes Alkylen, Alkenylen oder substituiertes Alkenylen mit 2 bis 20 Kohlenstoffatomen darstellt und A′ Alkylen oder substituiertes Alkylen mit 1 bis 18 Kohlenstoffatomen ist,
(c) 0,1 bis 10,0 Gew.Teile eines Keimbildners und
(d) 5 bis 60 Gew.Teile eines Verstärkungsmittels.

4. Zusammensetzung nach Anspruch 3, worin A, A′ und R′ jeweils Alkylen bedeuten und R Alkyl ist.

5. Zusammensetzung nach Anspruch 3, worin der Phthalimidoester einen Monophthalimidoester umfaßt, worin die Gesamtzahl von Kohlenstoffatomen von A und R 5 bis 30 Kohlenstoffatome ist.

6. Zusammensetzung nach Anspruch 3, worin der Phthalimidoester einen Diphthalimidoester umfaßt, worin die Gesamtzahl von Kohlenstoffatomen von A, R′ und A′ 4 bis 36 Kohlenstoffatome ist.

7. Zusammensetzung nach Anspruch 3, worin der Keimbildner ausgewählt ist aus dem Salz einer Dimersäure, dem Salz einer Trimersäure oder dem Salz einer Mischung einer Dimersäure und einer Trimersäure.

8. Zusammensetzung nach Anspruch 3, worin der Keimbildner ein Copolymer umfassend 85 Gew.% Äthylen und 15 Gew.% Methacrylsäure ist, das teilweise mit Natriumionnen neutralisiert wurde.

9. Zusammensetzung nach Anspruch 3, worin das Verstärkungsmittel Glasfasern sind.

10. Zusammensetzung nach Anspruch 3, die 0,1 bis 5 Gew.Teile eines Kettenverlängerungsmittels enthält.

11. Zusammensetzung nach Anspruch 10, worin das Kettenverlängerungsmittel ein Polyepoxid ist.

12. Zusammensetzung nach Anspruch 3, worin das Polyäthylenterephthalat eine Strukturviskosität von mindestens 0,6 aufweist.

13. Zusammensetzung nach Anspruch 3, worin das Polyäthylenterephthalat eine Mischung von Polyäthylenterephthalat und Polybutylenterephthalat umfaßt.

14. Zusammensetzung nach Anspruch 3, die weiters mindestens ein Additiv ausgewählt aus Schlagmodifikatoren, Strömungspromotoren, Farbstoffen, Flammschutzmitteln, Kopplungsmitteln und Stabilisatoren für thermische oxidative und Lichtstabilisierung in wirksamen Mengen umfaßt.

15. Polyesterformmasse umfassend

(a) 40 bis 95 Gew.Teile Polyäthylenterephthalat mit einer Strukturviskosität von mindestens 0,4 dl/g;

(b) 0,5 bis 5,0 Gew.Teile eines Keimbildners,

(c) 5 bis 60 Gew.Teile Glasfasern und

(d) 0,5 bis 15 Gew.Teile eines Weichmachers umfassend einen Phthalimidoester der Formel

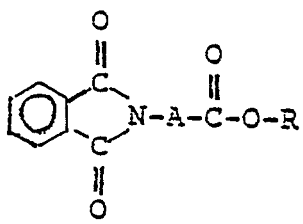

,

worin A Alkylen oder substituiertes Alkylen mit 1 bis 12 Kohlenstoffatomen bedeutet und R Alkyl oder Alkenyl mit 4 bis 20 Kohlenstoffatomen ist, oder

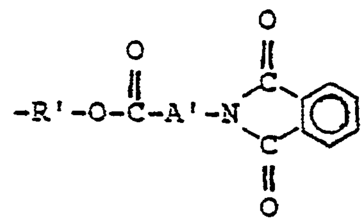

,

worin R′ Alkylen oder Alkenylen mit 2 bis 10 Kohlenstoffatomen bedeutet und A′ Alkylen mit 1 bis 12 Kohlenstoffatomen ist.

16. Polyesterformmasse umfassend

(a) 40 bis 95 Gew.Teile Polyäthylenterephthalat mit einer Strukturviskosität von mindestens 0,4 dl/g;

(b) 0,5 bis 5,0 Gew.Teile eines Keimbildners;

(c) 5 bis 60 Gew.Teile Glasfasern und

(d) 0,5 bis 15 Gew.Teile eines Weichmachers umfassend einen Phthalimidoester der Formel

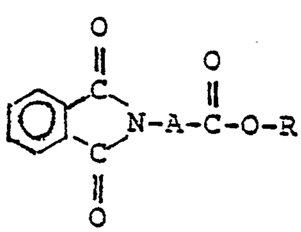

,

worin A Alkylen oder substituiertes Alkylen mit 1 bis 12 Kohlenstoffatomen darstellt und R Alkyl, substituiertes Alkyl, Alkenyl oder substituiertes Alkenyl mit 4 bis 20 Kohlenstoffatomen ist.

**Revendications**

1. Composition de polyester comprenant:
(a) un poly(téréphtalate d'éthylène) présentant un indice limite de viscosité d'au moins 0,4 décilitre/gramme mesuré sous forme d'une solution à 0,5% en poids dans un mélange 60:40 de phénol et de tétrachloroéthane à 25°C; et
(b) un plastifiant composé d'un phtalimidoester ayant pour formule:

$$X - A - \overset{O}{\overset{\|}{C}} - O - R$$

dans laquelle X représente un groupe phthalimido, dihydrophtalimido, tétrahydrophtalimido ou hexahydrophtalimido et A représente un groupe alkylène ou alkylène substuitué ayant de 1 à 18 atomes de carbone et R représente un groupe alkyle, alkyle substitué, alcényle ou alcényle substitué, ayant de 4 à 20 atomes de carbone, ou

$$- R' - O - \overset{O}{\overset{\|}{C}} - A' - X'$$

sachant que X′ est identique à X ou différent de X et représente un groupe phtalimido, dihydrophtalimido, tétrahydrophtalimido ou hexahydrophtalimido et que R′ représente un groupe alkylène, alcénylène, alkylène substitué ou alcénylène substitué ayant de 2 à 20 atomes de carbone et que A′ représente un groupe alkylène ou alkylène substitué ayant de 1 à 18 atomes de carbone.

2. Composition selon la revendication 1, dans laquelle le phtalimidoester est présent en une quantité d'au moins 0,1% en poids du poly(téréphtalate d'éthylène).

3. Composition de moulage de polyester comprenant:
(a) de 40 à 95 parties en poids d'un poly(téréphtalate d'éthylène) présentant un indice limite de viscosité d'au moins 0,4 décilitre/gramme mesuré sous forme d'une solution à 0,5% en poids dans un mélange 60:40 de phénol et de tétrachloroéthane à 25°C;
(b) de 0,1 à 15 parties en poids d'un plastifiant composé d'un phtalimidoester ayant pour formule:

$$X - A - \overset{O}{\overset{\|}{C}} - O - R$$

dans laquelle X représente un groupe phtalimido, dihydrophtalimido, tétrahydrophtalimido ou hexahydrophtalimido et A représente un groupe alkylène ou alkylène substitué ayant de 1 à 18 atomes de carbone et R représente un groupe alkyle, alkyle substitué, alcényle ou alcényle substitué ayant de 4 à 20 atomes de carbone, ou

$$- R' - O - \overset{O}{\overset{\|}{C}} - A' - X'$$

sachant que X′ est identique à X ou différent de X et représente un groupe phtalimido, dihydrophtalimido, tétrahydrophtalimido ou hexahydrophtalimido et que R′ représente un groupe alkylène, alkylène substitué, alcénylène ou alcénylène substitué ayant de 2 à 20 atomes de carbone et que A′ représente un groupe alkylène ou alkylène substitué ayant de 1 à 18 atomes de carbone;
(c) de 0,1 à 10,0 parties en poids d'un agent de nucléation; et
(d) de 5 à 60 parties en poids d'un agent de renforcement.

4. Composition selon la revendication 3, dans laquelle A, A′ et R′ représentent chacun un groupe alkylène et R représente un groupe alkyle.

5. Composition selon la revendication 3, dans laquelle le phtalimidoester se compose d'un monophtalimidoester dans lequel le nombre total d'atomes de carbone de A et de R est compris entre 5 et 30.

6. Composition selon la revendication 3, dans laquelle le phtalimidoester se compose d'un diphtalimidoester dans lequel le nombre total d'atomes de carbone de A, R′ et A′ est compris entre 4 et 36.

7. Composition selon la revendication 3, dans laquelle l'agent de nucléation est choisi parmi le sel d'un acide dimère, le sel d'un acide trimère ou le sel d'un mélange d'un acide dimère et d'un acide trimère.

8. Composition selon la revendication 3, dans laquelle l'agent de nucléation est un copolymère comprenant 85% en poids d'éthylène et 15% en poids d'acide méthacrylique que l'on a partiellement neutralisé avec des ions sodium.

9. Composition selon la revendication 3, dans laquelle l'agent de renforcement est constitué par des fibres de verre.

10. Composition selon la revendication 3, contenant de 0,1 à 5 parties en poids d'un extendeur de chaîne.

11. Composition selon la revendication 10, dans laquelle l'extendeur de chaîne est un polyépoxyde.

12. Composition selon la revendication 3, dans laquelle le poly(téréphtalate d'éthylène) présente un indice limite de viscosité d'au moins 0,6.

13. Composition selon la revendication 3, dans laquelle le poly(téréphtalate d'éthylène) se compose d'un mélange de poly(téréphtalate d'éthylène) et de poly(téréphtalate de butylène).

14. Composition selon la revendication 3, qui comprend encore au moins un additif choisi parmi des agents de modification de la résistance au choc, des agents améliorant la fluidité, des agents colorants, des agents ignifugeants, des agents de couplage et des stabilisants envers l'oxydation thermique et envers la lumière, en quantités efficaces.

15. Composition de moulage de polyester, comprenant:

(a) de 40 à 95 parties en poids de poly(téréphtalate d'éthylène) présentant un indice limite de viscosité d'au moins 0,4 décilitre par gramme;

(b) de 0,5 à 5,0 parties en poids d'un agent de nucléation;

(c) de 5 à 60 parties en poids de fibres de verre;

(d) de 0,5 à 15 parties en poids d'un plastifiant composé d'un phtalimidoester ayant pour formule:

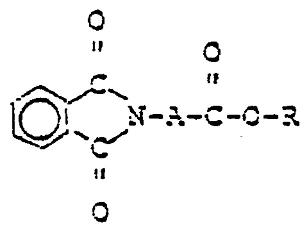

dans laquelle A représente un groupe alkylène ayant de 1 à 12 atomes de carbone et R représente un groupe alkyle ou alcényle ayant de 4 à 20 atomes de carbone, ou

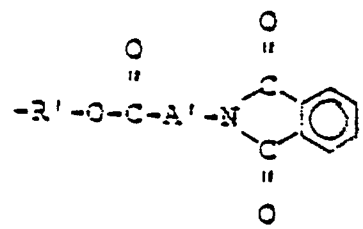

sachant que R′ représente un groupe alkylène ou alcénylène ayant de 2 à 10 atomes de carbone et que A′ représente un groupe alkylène ayant de 1 à 12 atomes de carbone.

16. Composition de moulage de polyester, comprenant:

(a) de 40 à 95 parties en poids de poly(téréphtalate d'éthylène) présentant un indice limite de viscosité d'au moins 0,4 décilitre par gramme;

(b) de 0,5 à 5,0 parties en poids d'un agent de nucléation;

(c) de 5 à 60 parties en poids de fibres de verre;

(d) de 0,5 à 15 parties en poids d'un plastifiant composé d'un phtalimidoester ayant pour formule:

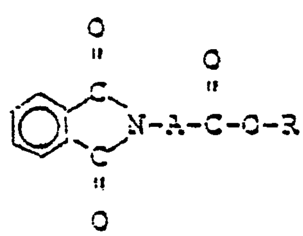

dans laquelle A représente un groupe alkylène ou alkylène substitué ayant de 1 à 12 atomes de carbone et R représente un groupe alkyle, alkyle substitué, alcényle ou alcényle substitué ayant de 4 à 20 atomes de carbone.